# NEW EUROPEAN PATENT SPECIFICATION

(11) **EP 1 511 466 B2**
(45) Date of publication and mention of the opposition decision: **25.02.2015**
(45) Mention of the grant of the patent: 08.10.2008
(21) Application number: 03704789.1
(22) Date of filing: 14.02.2003
(51) Int. Cl.: A61K 9/12, A61K 31/726, A61K 31/727, A61P 11/00, A61K 9/72, A61K 31/728

(54) **USE OF GLYCOSAMINOGLYCANS SUCH AS E.G. HEPARIN FOR THE TREATMENT OF RESPIRATORY DISORDERS SUCH AS COPD**
VERWENDUNG VON GLYKOSAMINGLYKANEN WIE Z.B. HEPARIN ZUR BEHANDLUNG VON ATEMERKRANKUNGEN WIE COPD
UTILISATION DE GLYCOSAMINOGLYCANES TELS QUE L'HEPARINE DANS LE TRAITEMENT D'AFFECTIONS RESPIRATOIRES TELLES QUE LA BPCO

(30) Priority: 18.02.2002 GB 0203830; 18.02.2002 GB 0203773; 17.05.2002 GB 0211414; 18.10.2002 GB 0224330
(43) Date of publication of application: 09.03.2005
(73) Proprietor: Ockham Biotech Limited, Swanwick Hampshire SO31 7HA (GB)
(72) Inventor: SHUTE, Janis Kay, Portsmouth, Hampshire P06 2QP (GB); CARROLL, Mary Patricia, Adult Cystic Fibrosis Unit, Tremona Road, Southampton SO16 6YD (GB); Dr.CONWAY, Joy H., Romesy , Hampshire,SO51 8DB (GB); HOCKEY, Peter Morey, Lymington, Hampshire SO41 5TB (GB)
(74) Representative: Woods, Geoffrey Corlett
(86) International application number: PCT/GB2003/000663
(87) International publication number: WO 2003/068187

(56) References cited:
- WO-A-00/25723
- WO-A-02/32406
- WO-A-99/06025
- US-A- 6 077 683
- RAO N V ET AL: "SULFATED POLYSACCHARIDES PREVENT HUMAN LEUKOCYTE ELASTASE-INDUCED ACUTE LUNG INJURY AND EMPHYSEMA IN HAMSTERS" AMERICAN REVIEW OF RESPIRATORY DISEASE, NEW YORK, NY, US, vol. 142, no. 2, August 1990 (1990-08), pages 407-412, XP001041125 ISSN: 0003-0805
- FROMMHERZ K ET AL: "EFFECT OF SULFATED GLYCOSAMINOGLYCANS ON THE INHIBITION OF NEUTROPHIL ELASTASE BY ALPHA1-PROTEINASE INHIBITOR" ADVANCES IN EXPERIMENTAL MEDICINE AND BIOLOGY, SPRING ST., NY, US, 1991, pages 161-165, XP001034168 ISSN: 0065-2598
- ANONYMOUS: "The definition of COPD" INTERNET ARTICLE, [Online] XP002242154 Retrieved from the Internet: <URL:www.priory.com/cmol/definiti.html> [retrieved on 2003-05-22]
- ANONYMOUS: "introduction : what is COPD ?" A GUIDE TO LIVING WITH COPD, [Online] XP002242155 Retrieved from the Internet: <URL:www.lung.ca/copd/intro/definition.htm l> [retrieved on 2003-05-22]
- MULLOY, B. ET AL.: 'Characterization of Unfractionated Heparin: Comparison of Materials from the last 50 Years' THROM. HAEMOST vol. 84, 2000, pages 1052 - 1056
- LEDSON, M. ET AL.: 'Nebulized heparin in Burkholderia cepacia colonized adult cystic fibrosis patients' EUR. RESPIR J. vol. 17, 2001, pages 36 - 38
- SHUTE, J. ET AL: 'abstract "Anti-inflammatory effects of inhaled nebulised heparin in adult CF patients-results of a pilot study' "THE WORLD'S KNOWLEDGE" 2000, page A75
- SALATHE, M. ET AL.: 'Treatment of Mucociliary Dysfunction' CHEST vol. 100, 1996, pages 1048 - 1057
- BARNES, PETER, J.: 'The cytokine network in asthma and chronic obstructive pulmonary disease' THE JOURNAL OF CLINICAL INVESTIGATION vol. 118, 2008, pages 3546 - 3556
- Global Initiative for Chronic Obstructive Lung Disease, National Institutes of Health, National Heart, Lung, and Blood Institute, publication number 2701, March 2001

## Description

### Field of Invention

The present invention relates to the manufacture of medicaments for treating chronic airflow limitation of the lung.

### Background of Invention

Chronic airflow limitation (CAL) is a common disease worldwide. The majority of individuals with CAL are smokers, or former smokers, and this is thought to be one of the primary causes of the condition. However, not all sufferers of CAL are smokers and it is also believed that exposure to pollutants and/or certain inhaled chemicals may also be a contributory factor in the development of CAL.

At present CAL is treated only in its more developed stages using a variety of bronchodilators such as β₂ agonists and anti-cholinergic agents. A proportion of those with CAL respond to steroids, such as glucocorticosteroids. The existing treatments for CAL are generally regarded as only partially effective and are not thought to halt the progressive decline in lung function. An unfortunate factor is that CAL is only diagnosed in its later stages, at which point the decline in lung function is already well advanced.

The only proven disease modifying intervention at present recognised for CAL is the cessation of smoking. Stopping smoking may only slow the progressive decline in lung function found in CAL. It is thought that inflammation may continue even after smoking is stopped and that the existing inflammation, and in particular inflammatory cells present in the lung, may cause the release of inflammatory mediators that prolong or even amplify the existing inflammation. In addition, stopping smoking is often difficult for sufferers with some not stopping at all or resuming smoking later on. On average less than one third of patients are able to give up cigarettes even with support.

US 6,077,683 is concerned with a method of synthesis of desulfated heparin and use thereof for inhibition of elastase and cathepsin.

Rao et al, (1990), American Review of Respiratory Disease, 142(2):407-412 refers to sulfated polysaccharides preventing human leukocyte elastase-induced acute lung injury and emphysema in hamsters.

Frommherz et al (1991), Advances in Experimental Medicine and Biology, 161-165, Springer press, refers to the effect of sulfated glycosaminoglycans on the inhibition of neutrophil elastase by alpha-1-proteinase inhibitor.

WO 99/06025 is concerned with methods and compositions for treating late phase allergic reactions and inflammatory diseases.

J. P. Boyle et al., Amer. J. Cardiol. (1964), vol. 14, 25-28 describes the use of intravenous heparin in the treatment of chronic obstructive pulmonary disease.

### Summary of the Invention

The present invention is based on the unexpected finding that glycosaminoglycans with an average molecular weight of from 8 to 40 kd can be used to treat CAL. The use of heparin is especially preferred. The use of administration by inhalation and/or intranasally is also particularly preferred.

Accordingly, the present invention provides for the use of a glycosaminoglycan or a physiologically acceptable salt thereof in the manufacture of a medicament for facilitating the clearance of mucus from the central and peripheral airways of a human subject with chronic airflow limitation (CAL) who has mucus hypersecretion wherein the said glycosaminoglycan or salt has an average molecular weight of from 12 to 18 kilodaltons and the medicament is administered via inhalation, intranasally, and/or instillation.

### Brief Description of the Figures

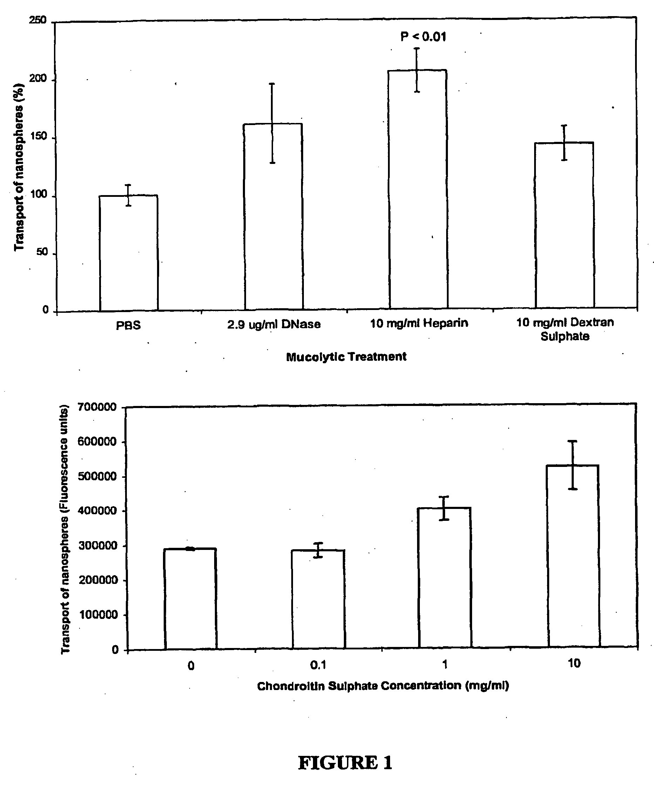

**Figure 1****: Effect of mucolytic agents on CAL sputum (n=3).** As described in Example 1, CAL sputum was homogenised and mucolytic added on a 10 % v/w basis. Mixing was performed using a 19-gauge needle. Carboxylate-modified fluorescent beads were added to sputum sample in a 1:1 ratio (v/w). After vortexing, 20 µl of sample was added to each upper well of a micro-boyden chamber. The upper and lower wells of the chamber were separated by an 8 µm polycarbonate filter, and the lower wells contained 25 µl PBS. The chamber was centrifuged for 5 mins at 1000 rpm to remove air bubbles prior to incubation at 37°C, 900 rpm, for 4 hours in the dark. Following incubation the chamber was dismantled and the fluorescence of the solution in the lower wells measured. The results for PBS, heparin, DNase and dextran sulphate (a non-glycosaminoglycan control) are shown in the top panel. The results for a mixture of chondroitin sulphates A and C are shown in the bottom panel.

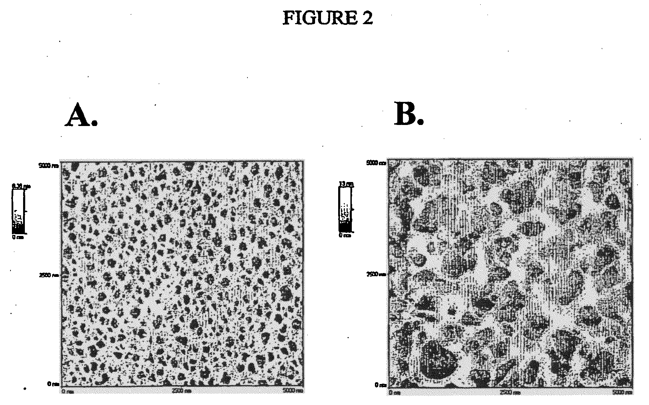

**Figure 2****: Atomic Force Microscopy (AFM) imaging of DNA.** As described in Example 2, calf thymus DNA (0.1 mg/ml) was treated with heparin (0.1 mg/ml) and AFM was performed in air under ambient conditions using a TopoMetrix TMX2000 Scanning Probe Microscope (ThermoMicroscopes, Bicester, UK). Panel A shows untreated DNA and Panel B shows DNA treated with heparin.

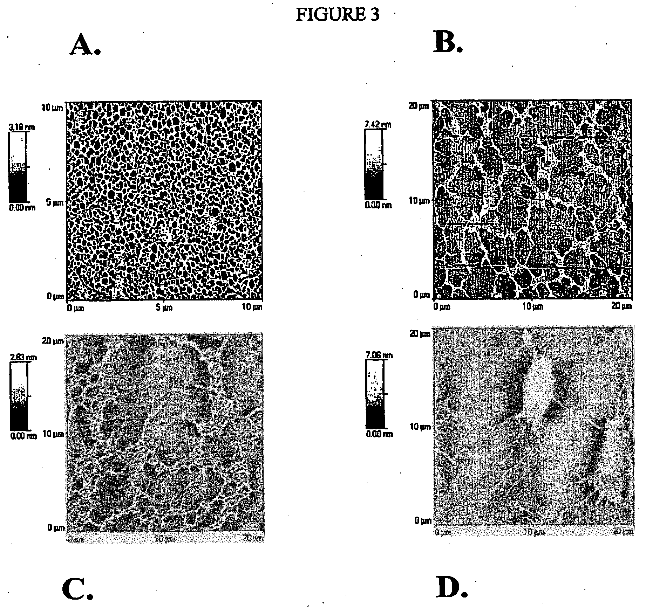

**Figure 3****: Atomic Force Microscopy (AFM) imaging of DNA.** Further Atomic Force Micrographs of DNA (100 µg/ml) treated with lower concentrations of heparin. Panel A shows the results for untreated DNA: Panel B for DNA treated with 0.1µg/ml heparin; Panel C for DNA treated with 1 µg/ml heparin; and Panel D for DNA treated with 10 µg/ml heparin.

### Detailed Description of the Invention

Chronic airflow limitation disorder (CAL) is a progressively dehabilitating condition which is often fatal. It is one of the most frequent causes of adult fatality. The present invention provides various medicaments for the treatment of CAL sufferers. These medicaments comprise a glycosaminoglycan, or a salt thereof. The glycosaminoglycan or salt will have an average molecular weight of from 12 to 18 kilodaltons.

### Subjects treated

The medicaments of the present invention are to be used to treat subjects suffering from, or at risk of developing, CAL. Typically, the subject will be a human but, as discussed further below, may be a vertebrate animal. CAL is a disease state characterized by airflow limitation that is not fully reversible. The airflow limitation is usually both progressive and associated with an abnormal inflammatory response of the lungs to noxious particles or gases. In particular, although not exclusively, CAL is associated with smoking.

For the purposes of the present invention CAL may be defined as a condition where there is a progressive decline in lung function, with the affected subject having an FEV₁ of less than 80% of that predicted for an individual of that age/race and/or height and/or who displays a FEV₁/FVC ratio of less than 70%. In an especially preferred embodiment, the subject to be treated will have an FEV₁ of less than 75% of that predicted.

Typically the reduction of FEV₁ is only partially reversible. In particular the reduction in FEV₁ is only partially reversible by treatment with bronchodilators such as, for example, β2 adrenergic agonists and in particular salbutamol.

FEV₁ is defined as the maximal forced volume which can be expired in one second starting from maximum inspiration (European Resp. Journal, 1993; 6: Suppl. 16 and Coates, Lung Function,: Assessment and Applications In Medicine, 4th Edition, Oxford, Blackwell, 1969). It can be measured by standard techniques well known in the art i.e. by spirometry. The FEV₁ for an individual may be from 10 to 80% of that predicted. Typically, the FEV₁ of the subject will be from 10 to 75% of the predicted value. Preferably the subject may have a FEV₁ of from 60 to 75% the predicted value, more preferably from 40 to 60% of predicted and even more preferably a value below 40% of that predicted. The subject may have an FEV₁ of less than 70%, preferably less than 60%, more preferably less than 50% and even more preferably less than 40% of that predicted. As discussed further below, the subject will typically have a history of exposure to pollutants or chemicals and in many cases will be, or have been, a tobacco smoker.

The FEV₁ value for the subject will normally be measured against predicted values and adjusted for age/sex/race and/or height. Predicted values may be those taken from Coates *(supra).* The expected value, which the value obtained for the subject may be compared to, may be the average expected value for smokers, or non-smokers, or both groups combined, preferably the expected value will be that for non-smokers not suffering from CAL (Coates, *supra*).

The reduction in FEV₁ in the subject will only be partially reversible and in particularly only be partially reversible on administration of a bronchodilator. Thus, for example, an increase in FEV₁ over the base-line value for the subject (i.e. that prior to administration of the bronchodilator) of more than 15%, preferably more than 20% and even more preferably over 25% will be regarded as reversibility. The increase may begin from 5 to 30, preferably from 10 to 25, more preferably over a period of from 15 to 20 minutes after the administration of the bronchodilator. Preferably the increases will begin from 15 minutes after the administration of the bronchodilator. The increases persist typically from 3 to 6 hours, preferably from 4 to 5 hours and more preferably 4 hours. Typically the bronchodilator used in assessing reversability will be a β₂ adrenergic agonist such as salbutamol, or ipratropium. In one embodiment of the invention the reduction in FEV₁ may be totally, or almost totally refractory to treatment with bronchodilators.

The subject may also show similar minimal increases in FEV₁ with steroid drugs such as Becotide^{™} or Prednisolone^{™} although typically response to such agents will not be used to define reversibility. The increases will also occur over a longer time period such as after 2 to 3 days and, if the steroid drugs are continually administered, persist. If steroids are stopped the improvement may persist for from 12 to 48 hours, or for days, weeks or even months, such as from six hours to six weeks, preferably from 1 day to 3 weeks.

Tests to assess reversibility of reduction of FEV₁ will typically be performed when the subject is clinically stable and free from infection. The subject should not have taken, or have had administered to them, inhaled short-acting bronchodilators in the previous six hours, long-acting β agonists in the previous 12 hours or sustained release theophyllines in the preceding 24 hours.

Spirometric values should typically be measured before and after an adequate dose of inhaled bronchodilator is given to the subject. The dose should preferably be selected to be high on the dose/response curve and usually will be given by nebuliser to be certain it has been inhaled. A similar dose may be given with multiple inhalations from a metered dose inhaler and large volume spacer, but this is less preferred. A typical dosage/measurements protocol for a human subject would be:
- before and 15 minutes after 2.5 to 5mg nebulised salbutamol or 5 to 10 mg terbutaline;
- before and 30 minutes after 500 µg nebulised ipratropium bromide; or
- before and 3 0 minutes after both in combination.

Equivalent protocols may be used for non-human subjects.

The FVC of the subject may also be measured in the diagnosis of CAL. The ratio of FEV₁ to FVC can be used in the diagnosis of CAL. Subjects to be treated will typically have an FEV₁ /FVC value of less than 70%. The ratio of FEV₁ /FVC may be below 65%, preferably below 60%, more preferably below 55% and even more preferably below 55%. In an especially preferred embodiment, a subject will have a FEV₁ /FVC of below 70% and also have a FEV₁ value of 80% or less of that predicted.

FVC (forced vital capacity) corresponds to the maximal volume of air forcibly exhaled from the point of maximal inhalation and can be measured using standard spirometry. In particular, the above specified values for FEV₁ /FVC will be those after administration of a bronchodilator as outlined above. The reduction in FEV₁ /FVC will typically show the same lack of reversibility as FEV₁.

Spirometric assessment is the most preferred method for diagnosing CAL and hence method for identification of subjects who may be treated. Accordingly, in an especially preferred embodiment of the invention spirometric assessment will be used in the diagnosis of a subject who has CAL and hence is treatable using the invention. In addition, the symptoms displayed by the subject may also be assessed to help confirm a diagnosis of CAL. Typically diagnosis will involve spirometric assessment in combination with assessment of the symptoms of a subject as well as elucidating whether the subject has a history of exposure to risk factors. In some situations spirometric assessment may not be possible, particularly in situations where resources are limited, and CAL will be diagnosed by alternative means such as by looking for the symptoms of CAL listed herein and a history of exposure to risk factors for CAL. Although chest X-rays are not typically indicative of whether or not a subject has CAL, they may be used to diagnose other respiratory disorders, such as TB, and hence rule out CAL.

The subject will show, or have previously shown, an accelerated rate of decline of lung function compared to the average expected for an equivalent individual not suffering from CAL and in particular for an equivalent non-smoking individual. The subject may display shortness of breath and in particular may do so after physical exertion such as on exercise. Typically, this will not be induced by exposure to an allergen. The subject may also show increased incidence of bacterial or viral infection and this may exacerbate the condition.

The individual may show a rate of decrease in FEV₁ one, two, three, four or more times greater than the average annual value expected for an equivalent individual not suffering from CAL. For instance a subject over thirty may show an annual reduction of from 50 to 100, preferably from 50 to 80 and more preferably from 60 to 70 ml FEV₁/yr compared to a reduction of from 10 to 40 and typically of 20 to 40 ml of FEV₁/yr in the equivalent non-smoker. These values may also apply to non smoking sufferers of CAL such as where the disorder is cased by pollutants.

Subjects with CAL may display one or more, and sometimes all, of cough, increased sputum production, dyspnea, and/or a history of exposure to risk factors for the disease. In the case of cough, increased sputum and dyspnea these may have been present for extended periods of time such as at least a month, preferably six months, more preferably at least a year and still more preferably for at least two years. Chronic cough and sputum production often precede the development of CAL and may be indicative of individuals for which the invention can be used prophylactically to prevent the development of CAL.

The subject may have a history of exposure to pollutants and/or chemicals and in particular to those in a form which can be inhaled. In particular, the subject may be a tobacco smoker or a former tobacco smoker. Typically, the subject may be, or have been, a heavy smoker, smoking from 10 to 100, preferably from 20 to 60, more preferably from 25 to 50 and even more preferably from 30 to 40 cigarettes per day. The subject may have done so for several years such as from 5 to 50, preferably from 10 to 40, more preferably from 15 to 30 and even more preferably from 20 to 25 years. The subject may typically have a smoking history of from 1 to 20, preferably 21 to 40, more preferably 41 to 60 pack years. They may have a smoking history, for example, of from 1 to 50, preferably from 10 to 40, more preferably from 20 to 30 pack years.

The individual may have been, or be, a pipe or cigar smoker. The individual may have chewed tobacco or products containing tobacco. In some cases the individual may have been passively exposed to tobacco smoke rather than smoke themselves. Thus the subject may, for example, have been cumulatively exposed to passive tobacco smoke for long periods due to their work, leisure and/or home environments. The subject may use inhaled drugs such as, for example cannabis or other drugs commonly mixed with tobacco before smoking.

The subject will typically be a mature adult. For example, the subject may be from 21 to 85, preferably from 25 to 70, more preferably from 30 to 60 and even more preferably from 40 to 50 years of age. The onset of any of, or a particular, symptom mentioned herein, will typically have been in adulthood. For example, the subject may have been at least 20, more preferably at least 25, still more preferably at least 30 and even more preferably at least 35 years of age before they experienced a particular symptom. In particular, the symptoms associated with more advanced stages of CAL, such as any of those mentioned herein, may have their onset at such later stages of life. Subjects with a genetic predisposition to developing CAL, such as those with α₁-antitrypsin deficiency, may develop CAL earlier. For example, they may display one or more, or a particular, symptom at from 10 to 21, preferably from 12 to 18, or more preferably from 14 to 16 years of age. Alternatively, they may first show the symptom at any of the age ranges mentioned herein. The subject may have been diagnosed at any of the ages, or within any of the age ranges, specified herein.

The subject may have additionally been, or alternatively been, exposed to other chemical or environmental pollutants. The subject may have been exposed to occupational dusts and chemicals (vapours, irritants and fumes) and/or indoor or outdoor air pollution. The subject may have a history of exposure to particulate matter, irritants, organic dusts and sensitising agents. The subject may work, or have worked, in an environment which exposes them to chemicals and/or pollutants. Thus, for example, the subject may be a factory worker or miner such as a coal miner. The subject may cook in kitchens with poorly ventilated cooking stoves such as in the presence of kerosene cookers. This, in particular, may be the case in developing countries such as Africa. The subject may work in the construction industry. The subject may have been exposed to high levels of pollution, such as exhaust emissions from vehicles or other engines. The subject may have been exposed to smog or sulphur dioxide containing emissions. The individual may live in the city centre. The individual may live in close proximity to heavy industry.

The subject may have a genetic predisposition to developing CAL and may display a family history of the condition. For example, the subject may have α₁-antitrypsin deficiency and hence be predisposed to developing CAL. Subjects at risk of developing CAL may have had low birth weights and/or a history of exposure to pollutants in the womb or in early life. The mother of the subject may be a smoker and may have continued to smoke during pregnancy. The subject may have had a history of severe childhood respiratory infection. Reduced maximal attained lung function, as measured by spirometry, may identify individuals who are at increased risk of developing CAL.

The subject may have an early stage of CAL in which the symptoms are generally moderate and may have periods of normality or of at least reduced symptoms. Alternatively, the subject may have a more developed stage of CAL in which the symptoms, and particular the reduction in FEV₁ is more pronounced. Preferably, the medicaments of the invention are used, or administered at, an early stage of CAL so that they can arrest, slow or regress the increased rate in FEV₁ decline at as early a stage as possible.

CAL is typically a progressive disorder with the severity of CAL and the extent to which it has an impact on the sufferer increasing over time. Thus there may be a progressive manifestation in the symptoms of the disorder. Chronic cough is usually the first symptom to develop. It may initially be intermittent, but later may be present every day. The cough will typically be present throughout the day, rather than just at night and in the morning. In some cases, significant airflow limitation may develop without the presence of a cough. Small amounts of tenacious sputum are commonly raised after coughing bouts.

As the disease progresses the subject may experience dyspnea. The onset of dyspnea will often be one of the reasons why a human subject will first consult a physician as it can be dehabilitating and also induce anxiety. As the lung function of the subject deteriorates further, breathlessness becomes more intrusive. The subject may display wheezing and chest tightness. The dyspnea may become worse during or after exercise. Respiratory infections may also exacerbate the condition and cause increased dyspnea. Human subjects may indicate that the dyspnea progressively impairs their ability to carry out physical labour and to exert themselves.

CAL sufferers are sometimes split into categories which reflect the stage and severity of the disease. This can help define the needs of a particular subject and what course of treatment should be administered. In one classification (GOLD Executive Summary, supra) subjects are split into the following categories:

### Category 0 - At risk

Lung function, as measured by spirometry, is normal.
Chronic symptoms (cough, sputum, production).
Typically, human subjects will be unaware of abnormal lung function.

### Category I: Mild CAL

Mild airflow limitation.
FEV₁/FVC < 70%.
FEV₁ greater than, or equal to, 80% of that predicted.
With or without chronic symptoms (cough, sputum, production).
Human subjects may be unaware that their lung function is abnormal.

### Category II: Moderate CAL

Worsening airflow limitation.
FEV₁/FVC < 70%.
FEV₁ from 30 to 80% of that predicted (category can be subdivided into: IIA - FEV₁ from 50 to 80% of that predicted; and IIB - FEV₁ from 30 to 50% of that predicted). With, or without, chronic symptoms (cough, sputum, production, dyspnea). The symptoms may typically become more pronounced on exertion. Human subjects are likely to be aware of the condition and have sort medical advice.

### Category III: Severe CAL

Severe airflow limitation.
FEV₁/FVC < 70%
FEV₁ less than 30% predicted or alternatively an FEV₁ less than 50% of that predicted in conjunction with respiratory failure or clinical signs of right heart failure (respiratory failure: arterial partial pressure of oxygen (PaO₂) less than 8.0 kPa (60 mm Hg) with or without arterial partial pressure of CO₂ (PaCO₂) greater than 6.7 kPa (50 mm Hg) while breathing air at sea level).

The subject to be treated using the invention may fall within any of the above categories. In particular, the subject may be one in categories I to III, preferably in categories II to III, and even more preferably in category III. The invention also encompasses the treatment of individuals at risk of CAL (category 0) and with an early stage of the disorder (category I).

CAL is typically characterized by chronic inflammation throughout the airways, parenchyma, and pulmonary vasculature. Macrophages, T lymphocytes (predominately CD8⁺) and neutrophils may be present in increased levels in various parts of the lung. Typically, the condition will be characterised by the presence of large numbers of infiltrating neutrophils rather than eosinophils. Activated inflammatory cells release a variety of mediators, including leukotriene B4 (LTB4), interleukin 8 (IL-8), tumor necrosis factor (TNF) and others, and any of these may be present in elevated levels in a subject in comparison to healthy individuals who do not have CAL. In particular those mediators capable of causing damage to lung structure and/or sustaining neutrophilic inflammation will be present in elevated levels. The subject may also display an imbalance ofproteinases and anti-proteinases in the lung and also elevated oxidative stress.

The subject may also display increased inflammation. There may be elevated levels of inflammatory mediators present in the airways of the subject. These may include proteases such as matrix metallo proteases (MMPs), cathepsins and elastase. They may also include myelo-peroxidase, activated complement, MCP-1, interferons (such as, in particular, IFN-γ) and interleukins such as, in particular, IL-12. Typically, IL-8 and in particular elevated IL-8 levels will be present.

The lungs of the subject, or regions of them, may have increased numbers of inflammatory cells in comparison to those of an equivalent individual without CAL. In particular, there may be an influx of neutrophils and/or CD8⁺ T cells into the airways and lung. There may also be changes in the bronchial and pulmonary vasculature, such as upregulation of adhesion molecules involved in leukocyte extravasation or increased expression of inflammatory cytokines which can recruit inflammatory cells such as neutrophils, monocytes and T cells.

The neutrophils may be primed or in an activated state. For example, they may show an increase in expression of various adhesion molecules and receptors. There may also be an elevated level of neutrophil degranulation and hence of neutrophil proteins in the lungs. These may include proteins such as proteases (including cathepsins and neutrophil elastase), other hydrolytic enzymes and various antibacterial proteins. There may also be an elevation in the level of reactive oxygen species released by the neutrophils. These changes may be detectable in the subject by examining samples such as sputum or lavages from the subject. There may also be elevated levels of other granulocytes present in the inflamed airway and lung such as basophils and/or eosinophils, but typically these will not be the predominate species of granulocytes.

In some of the subjects there will be permanent destructive enlargement of the airspaces distal to the terminal bronchioles without obvious fibrosis. There may be chronic hypersecretion of mucus. Thus mucus secretion may, for example, be increased one, two, three or four times. The viscosity of the mucus may also be increased and this may be at least partially due to the presence of polymers such as DNA and actin. The chronic bronchial secretions may in some cases be such that they are enough to cause expectoration, occurring on most days for a minimum of three months of the year for two consecutive years.

In some cases, there may be extracellular DNA, and in particular endogenous extracellular DNA, present in the lungs of the subject, including in the airways of the lungs. The DNA may be extracellular DNA originating from the cells of the body of the subject. Typically this DNA will originate from the nucleus and hence be genomic DNA. The DNA may originate from infiltrating inflammatory cells.

In cases where extracellular DNA is responsible for, or contributes to, an increase in mucus viscosity in the subject, it will typically be of high molecular weight. Thus it may be, for example, that it has an average fragment size, fragment size range or predominate fragment size in the range of from 100 bases to 1 megabase, preferably from 1 kb to 500 kb, more preferably from 5 kb to 250 kb in length, still more preferably from 25 kb to 100 kb and even more preferably from 25 kb to 50 kb in length. The DNA will typically be wholly, or partially free of, histones or will comprise regions which lack histones. It may be bound by cationic factors or factors with cationic groups such as inflammatory mediators and/or proteases. Thus for example it may be complexed or associated with elastase, cathepsins and/or IL-8. The presence of the DNA will typically increase the viscosity of the mucus, or other secretion, that it is present in.

Typically, the presence of DNA may increase the viscosity of the mucus by from 10 to 5000%, preferably from 50 to 2500%, more preferably from 100 to 1000%, still more preferably from 200 to 800% and even more preferably from 400 to 600%. The viscosity of the solution may typically be doubled, tripled, quadrupled or increased by five fold or more in comparison to the viscosity of the solution in the absence of the DNA. The viscosity of the DNA may be such that it may compromise a function in the subject such as, for example, lung function. It may contribute to airflow limitation. It may promote the occurrence of infections.

Pathological changes characteristic of CAL may typically be found in the central airways, peripheral airways, lung parenchyma and/or pulmonary vasculature of the subject. In the central airways (the trachea, bronchi and bronchioles greater than 2 to 4 mm in internal diameter) infiltrating inflammatory cells may be present in the surface epithelium. Enlarged mucus secreting glands and an increase in the number of goblet cells may also be seen and are typically associated with mucus hypersecretion. In the peripheral airways (small bronchi and bronchioles that have an internal diameter of less than 2 mm) chronic inflammation may lead to repeated cycles of injury and repair of the airway wall. The repair process typically results in remodeling of the airway wall, with increasing collagen content and scar tissue formation, that narrows the lumen and produces fixed airways obstruction.

Destruction of the lung parenchyma in subjects with CAL typically occurs as centrilobular emphysema. This involves dilatation and destruction of the respiratory bronchioles. These lesions occur more frequently in the upper lung regions in milder cases, but in advanced disease they may appear diffusely throughout the entire lung and also involve destruction of the pulmonary capillary bed. An imbalance of endogenous proteinases and antiproteinases in the lung, which may be due to genetic factors or the action of inflammatory cells and mediators, may be a major mechanism in the emphysematous lung destruction. Oxidative stress, another consequence of inflammation, may also play a part in the destruction.

The subject may display pulmonary vascular changes, with thickening of the vessel wall that begins early on in the development of the disease. Thickening of the intima is the first structural change, followed by an increase in smooth muscle and the infiltration of the vessel wall by inflammatory cells. As the severity of the disease increases, greater amounts of smooth muscle, collagens and proteoglycans may increase the thickness of the vessel wall further.

The lungs of the subject may display a number of physiological changes characteristic of CAL, including mucus hypersecretion, ciliary dysfunction, airflow limitation, pulmonary hyperinflation, gas exchange abnormalities, pulmonary hypertension, and cor pulmonale. Often such abnormalities will manifest themselves in that order, although they may not necessarily do so. Mucus hypersecretion and ciliary dysfunction may contribute to chronic cough and sputum production. These symptoms can be present for many years before other symptoms or physiological abnormalities develop and may be used to detect individuals who have a very early stage of CAL or are at risk of the disorder.

In more advanced stages of CAL, peripheral airways obstruction, parenchymal destruction, and pulmonary vascular abnormalities reduce the capacity of the lung for gas exchange, producing hypoxemia and, eventually, hypercapnia. Pulmonary hypertension, which develops late in the course of CAL when the disorder has reached its severe stage, is the major cardiovascular complication of CAL and may be associated with the development of cor pulmonale and a poor prognosis.

The subject will be a vertebrate animal and preferably will be a mammal. Typically the subject will be human. However, the invention also encompasses the manufacture of medicaments for the treatment of animals with conditions which are the same as, or equivalent to, CAL. Thus the animal condition may have either an underlying pathology similar to, or the same as, that of CAL in humans. The animal may have one or more similar symptoms or characteristics of human CAL and in particular one or more of those listed above. Preferably, the animal will suffer from a condition which falls within the definition of CAL given above.

In cases where the subject is not human it may be a domestic animal or an agriculturally important animal. The animal may, for example, be a sheep, pig, cow, bull, poultry bird or other commercially farmed animal. In particular, the animal may be a cow or bull and preferably is a dairy cow. The animal may be a domestic pet such as a dog, cat, bird, or rodent. In a preferred embodiment the animal may be a cat or other feline animal. The animal may be a monkey such as a non-human primate. For example, the primate may be a chimpanzee, gorilla, or orangutan. In a preferred embodiment of the invention the animal may be a horse and, for example, may be a racehorse. The animal may be a sports animal.

Some of the symptoms of CAL are displayed by sufferers of other diseases. For example, a number of respiratory disorders other than CAL compromise lung function. However, these diseases may be distinguished from CAL by a full assessment of the subject and preferably by applying the relevant available guidelines for the diagnosis of CAL. The British Thoracic Society have published a set of Guidelines (Thorax 1997, 52 (Suppl. 5): S1-28) and in an especially preferred embodiment a subject to be treated will fall within the definition of the disorder provided by these Guidelines. In addition, the Global initiative for chronic Obstructive Lung Disease (GOLD) has published an Executive Summary (2001) outlining a strategy for the diagnosis; management and prevention of the disorder. In a preferred embodiment the subjects to be treated will fall within the definition of the disorder provided by the Executive Summary.

CAL does not include cystic fibrosis (CF). Thus typically a subject who can be treated using the invention will have a least one functional copy of the CFTR gene (unless they have been unfortunate enough to develop both CF and CAL). CAL typically has a much later onset than Cystic Fibrosis, where a sufferer is born with the defect. In CAL the decline in lung function and the onset of symptoms will occur progressively over time. In CF the decline in lung function will have a more immediate onset, earlier on in life. A subject who has CAL will often be in their thirties, forties or even fifties before they become aware that they are suffering from the disorder. The major exception to the later onset of CAL, is in those sufferers who have α₁ anti-trypsin deficiency. Such subj ects will display an earlier onset of CAL, such as in their teens or twenties when CAL is diagnosed, as they will be born with a genetic condition predisposing them to CAL. Subjects who have α₁ antitrypsin deficiency and CF can readily be distinguished from each other by biochemical and genetic tests to identify the nature of the disorder.

### Glycosaminoglycans

The medicaments of the invention employ glycosaminoglycans. Glycosaminoglycans are linear heteropolysaccharides possessing characteristic disaccharide repeat sequences that are typically highly N-and O-sulpated at D-glucosamine, galatactosamine and uronic acid residues. These sulphate moieties introduce a high degree of negative charge along the glycosaminoglycan polymer chain and add to the heterogeneity of these macromolecules.

Any suitable glycosaminoglycan may be employed in the invention. Glycosaminoglycans and glycosaminoglycan salts suitable for use in the present invention will have an average molecular weight of from 12 to 18 kd. In particular, the glycosaminoglycan or salt may have an average molecular weight of from 12 to 18 kd. In some embodiments all, or substantially all, of the glycosaminoglycan molecules or glycosaminoglycan salt molecules will have a molecular weight falling within the ranges specified above. Thus from 50 to 100%, preferably from 75 to 100%, more preferably from 90 to 100%, still more preferably 95 to 100% of the molecules may have such a molecular weight. In some cases at least 95%, preferably 97.5%, more preferably 99%, still more preferably 99.5% and even more preferably 99.9% may have a molecular weight falling within the range. The glycosaminoglycan or salt may be present in a range of molecular weight sizes and typically the most commonly occurring molecular weight size will fall within one of the above specified molecular weight ranges.

Preferably, the glycosaminoglycan employed in the invention will be any of chondroitin sulphates A to E, heparin, heparin sulfate, heparan, heparan sulphate, hyaluronic acid, keratan sulphate, a derivative of any thereof or a mixture or any two thereof. Chondroitin sulphate B is sometimes referred to as dermatan sulphate. In a more preferred embodiment of the invention the glycosaminoglycan will be any of chondroitin sulphates A, C, D or E, heparin, heparin sulfate, heparan, heparan sulphate, hyaluronic acid, keratan sulphate, a derivative of any thereof or a mixture of any two thereof. In a particularly preferred embodiment the glycosaminoglycan will be chondroitin sulphate A, chondroitin sulphate C, heparin, heparin sulfate, heparan, heparan sulphate, a derivative of any thereof or a mixture of any two thereof. More preferably, the glycosaminoglycan will be chondroitin sulphate A, chondroitin sulphate C, heparin, a derivative of any thereof or a mixture of any two thereof. In an even more preferred embodiment of the invention the glycoaminoglycan will be heparin or a derivative thereof. In some embodiments of the invention the glycosaminoglycan employed will be a mixture of more than two glycosaminoglycans from one of the above mentioned groups, such as a mixture of three, four or five of the glycosaminoglycans.

In embodiments of the invention where a mixture of two glycosaminoglycans is employed the two may, for example, be present in the ratio 1:1, 1:2, 1:4, 1:10 or 1:100. The ratio may be 90:10, 80:20, 70:30, or 60:40. Any suitable ratio may be employed and either glycosaminoglycan may be at the higher concentration. The ratio may be the same as the ratio in which the two are isolated when they are recovered from a common tissue using standard techniques. In a preferred embodiment of the invention a mixture of chondroitins A and C will be employed and in particular at a ration of 80:20, preferably 75:25 and even more preferably 70:30 with chondroitin sulphate A being present at the higher level.

Typically, the glycosaminoglycan will not have been subjected to fragmentation to reduce its molecular weight. Usually, the glycosaminoglycan will not have been subjected to depolymerisation, such as by chemical or enzymatic means, to reduce its molecular weight. The average number of saccharide units in the polysaccharide chains of the glycosaminoglycan may typically be from 18 to 100, preferably from 30 to 80, more preferably from 40 to 60 and still more preferably from 5 to 60 units.

The glycosaminoglycan may be any suitable commercially available glycosaminoglycan and may, for example, be an unfractionated glycosaminoglycan. The glycosaminoglycan will have typically been isolated from a natural sources such as from an animal. In some cases, the glycosaminoglcan may have been synthesised rather than be a naturally occurring molecule.

In some cases the glycosaminoglycan may have been isolated from an animal, and in particular from animal tissues such as those of pigs or cattle. The glycosaminoglycan may have been obtained from tissues such as the lung, liver, or gut of an animal and in particular from beef lung or pork intestinal mucosa. The glycosaminoglycan may have been obtained from the skin of such an organism.

In some embodiments, the glycosaminoglycan may have been isolated from a cartilagenous fish or other sea or freshwater organism. In some cases the glycosaminoglycan may have been isolated from a shark or squid and in particular from the cartilage of such an organism. The glycosaminoglycan may have been isolated from a sturgeon and in particular from a sturgeon notochord.

One of the specific glycosaminoglycans mentioned above may have been modified to generate a derivative of the glycosaminoglycans. Thus such derivatives may be used in the invention as long as they retain therapeutic activity in treating CAL and in particular are capable of eliminating, reducing, ameliorating or managing one or more of the symptoms and manifestations of CAL discussed herein. Thus in the case of heparin, the heparin may have been subjected to O-desulphation such as at least at the 2-O and 3-O positions. The same or equivalent modifications may be made to other glycosaminoglycans to generate derivatives for use in the present invention.

The glycosaminoglycan may have been subjected to acetylation, deacetylation, oxidation and/or decarboxylation such as, for example, periodate oxidation to generate a derivative. Heparinoids may be used in the invention.

Typically, the active compound used in the present invention comprises is a glycosaminoglycan or a physiologically acceptable salt thereof comprising repeating disaccharide units of general formula (1)

-[A-B]- (1)

wherein:
each A is the same or different and represents a moiety of formula (i) or (ii) 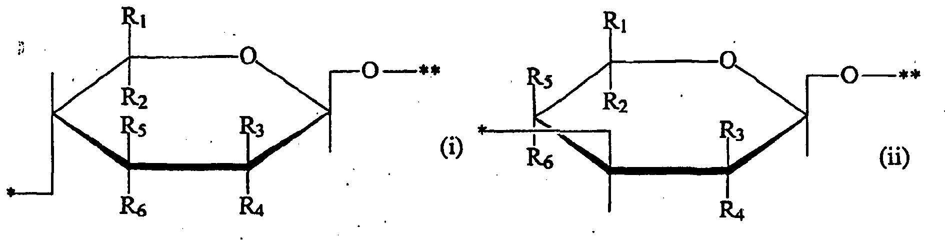
wherein:
- one of R₁ and R₂ is hydrogen, and the other is -CO₂H, -SO₃H or -CH₂OR wherein R is hydrogen or -SO₃H;
- one of R₃ and R₄ is hydrogen, and the other is -OR wherein R is hydrogen or -SO₃H;
- one of R₅ and R₆ is hydrogen, and the other is -OH;
- * represents a direct bond to an adjacent hydrogen atom or B moiety; and
- ** represents a direct bond to an adjacent B moiety;
each B is the same or different and represents a moiety of formula (iii) or (iv); 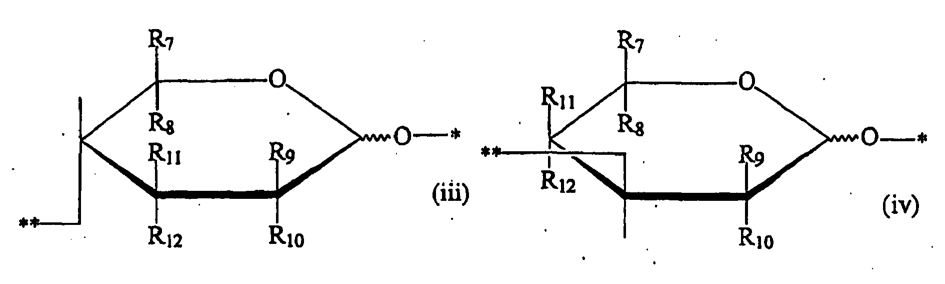 wherein:
- one of R₇ and R₈ is hydrogen and the other is -CH₂OH or -CH₂OSO₃H;
- one of R₉ and R₁₀ is hydrogen and the other is -NHAc, -NH₂ or -NHSO₃H;
- one of R₁₁ and R₁₂ is hydrogen and the other is -OH or -OSO₃H;
- * represents a direct bond to a hydrogen atom or an adjacent A moiety;
- ** represents a direct bond to an adjacent A moiety; and
- indicates a bond in either stereochemical orientation;
or a physiologically acceptable salt thereof.

The formulae herein adopt standard practice in depicting sugars. According to this practice, the formulae include vertical lines through each of the cyclic carbon atoms. This does not, of course, mean that methyl groups are attached at each position, or that methylene groups are present as part of the link between adjacent cyclic moieties.

Preferably, each A moiety in the glycosaminoglycan of general formula (1) is the same. Preferably, each B moiety in the glycosaminoglycan of general formula (1) is the same.

Preferably, each A moiety in the glycosaminoglycan of general formula (1) is a moiety of general formula (i).

Typically, one of R₁ and R₂ is hydrogen, and the other represents -CO₂H or -CH₂OR, wherein R is hydrogen or -SO₃H. Preferably, one of R₁ and R₂ is hydrogen and the other represents -CO₂H.

Typically, R₃ is hydrogen and R₄ is -OR, wherein R represents hydrogen or -SO₃H.

Typically R₅ is -OH and R₆ is hydrogen.

Typically each A is the same or different and represents a moiety of formula (i).

Typically R₇ is -CH₂OH or -CH₂OSO₃H and R₈ is hydrogen.

Typically R₉ is hydrogen and R₁₀ is -NHAc, -NH₂ or -NHSO₃H.

Typically R₁₁ is -OSO₃H or -OH and R₁₂ is hydrogen.

Typically each B is the same or different and represents 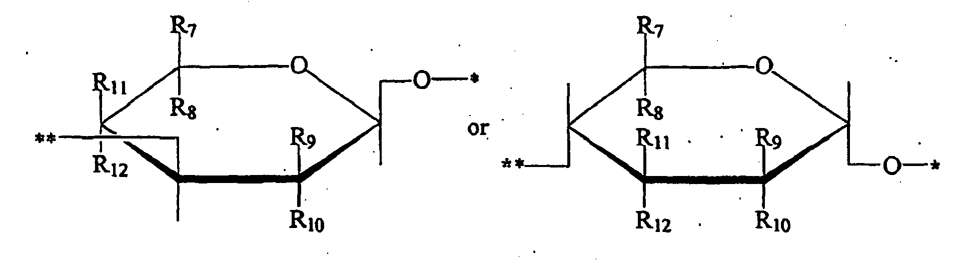 wherein R₇, R₈, P₉, R₁₀, R₁₁, R₁₂ * and ** are as described above.

Preferably R₁ is not hydrogen in an A moiety which is adjacent to a moiety (iv) in which R₁₂ is hydrogen.

Typically the glycosaminoglycan of general formula (1) is a glycosaminoglycan of general formula (2) 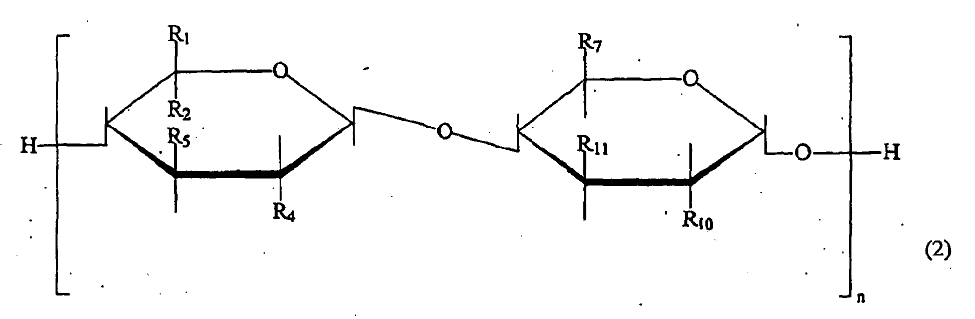 wherein:
- one of R₁ and R₂ is hydrogen and the other is -CO₂H;
- R₄ is -OH or -OSO₃H;
- R₅ is -OH;
- R₇ is -CH₂OH or -CH₂OSO₃H;
- R₁₀ is -NH₂, -NHSO₃H or -NHAc; and
- R₁₁ is -OSO₃H or -OH.

Typically the glycosaminoglycan of general formula (1) is a glycosaminoglycan of general formula (3) 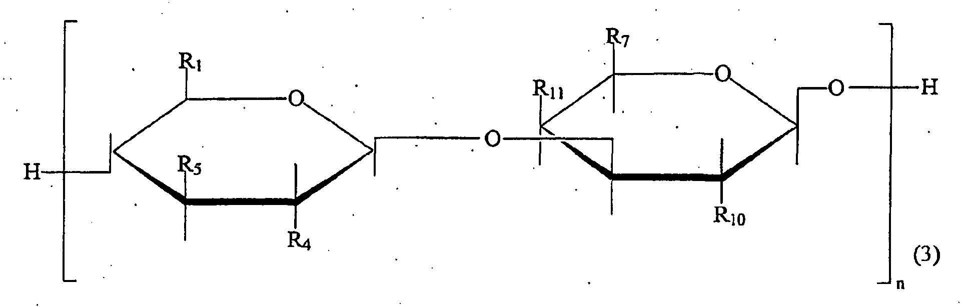 wherein:
- R₁ is -CO₂H;
- R₄ is -OH;
- R₅ is -OH;
- R₇ is -CH₂OH or -CH₂OSO₃H;
- R₁₀ is -NHAc; and
- R₁₁ is -OH or -OSO₃H.

Any suitable physiologically acceptable glycosaminoglycan salt may be employed in the invention and in particular a metallic salt, for example a sodium salt, an alkali metal or an alkaline earth metal salt. Other salts include calcium, lithium and zinc salts. Ammonium salts may also be used. The salt may be a sodium glycosaminoglycanate or glycosaminoglycan sulphate. Salts of derivatives of specific glycosaminoglycans mentioned herein may also be used in the invention. In the present application where mention of a glycosaminoglycan is made, such mention also includes physiologically acceptable salts thereof.

Typically a physiologically acceptable salt is a salt with a physiologically acceptable acid or base. Preferred salts are salts with physiologically acceptable bases. Typically, such salts are compounds wherein the acidic hydrogen atom of a -CO₂H and/or -OSO₃H group is replaced with a cation, for example an alkali metal (e.g. sodium or potassium) or alkali earth metal (e.g. calcium or magnesium) cation. Such salts can be prepared, for example, by reaction with an appropriate hydroxide.

The number of disaccharide units present in the glycosaminoglycan or salt thereof employed in the invention will be such that the molecular weight of the glycosaminoglycan or salt is from 12 to 18 kd. In particular, it may be such that the glycosaminoglycan has a molecular weight of from 14 to 18 kd, preferably from 15 to 17 kd and more preferably from 16 to 17 kd. The number of disaccharide units present in the glycosaminoglycan may be represented by the number n, where n is any integer such that the glycosaminoglycan has a molecular weight falling within any of the above mentioned molecular weight ranges.

Thus the glycosaminoglycan or salt employed may be represented by the general formula:

H-[A-B]ₙ-H

wherein A-B is any of the disaccharide units mentioned above and n is an integer such that the glycosaminoglycan or salt thereof has a molecular weight falling within the above specified molecular weight ranges. The value of n may be, for example, from 30 to 55 and more preferably from 35 to 50.

The glycosaminoglycan employed in the invention will typically comprise more than one length chain. Hence n for some of the glycosaminoglycan chains present may be a lower or higher integer than an integer which, on its own, would give a chain of molecular weight size falling within one of the above specified ranges. Thus the average value of n of the glycosaminoglycans present in the medicaments of the invention may be any of the values specified for n herein and in particular a value of n which gives a molecular weight glycosaminoglycan or salt falling within one of the molecular weight ranges specified herein.

Particularly preferred salts for use in the invention are salts of formula:

H-[A-B]ₙ^{x-}-H M^{x+}

wherein x≤4n and M represents a physiologically acceptable cation or a mixture thereof. Most preferably, x ≤2n.

In a particularly preferred embodiment of the invention the glycosaminoglycan employed will be a heparin, derivative thereof or a physiologically acceptable salt thereof. Heparin is a naturally occurring mucopolysaccharide present in a variety of organs and tissues, particularly liver, lung, and the large arteries. Heparin is a polymer of alternating α-D-glucosamine and hexuronate residues joined by (1,4) glycosidic linkages. When glycosaminoglycans are synthesised in nature, typically they are conjugated to a central protein core. However, preferably the glycosaminoglycans employed in the invention will lack such a central core. Typically, glycosaminoglycan preparations will lack a core and may be employed or, if present, the core can be removed. Commercially available preparations of glycosaminoglycans will usually lack the core and may be employed.

Heparin is clinically used as an anti-coagulant, where it is thought to exert its effects through interaction with anti-thrombin III (AT-III) and heparin co-factor II and other coagulation factors. Typically the heparin will retain some anticoagulant activity i.e. be able to increase clotting time in an individual. Thus preferably the heparin will be able to bind anti-thrombin III (AT-III) and/or heparin co-factor II (HCII) and hence inhibit clotting. Preferably it will be able to form a complex with AT-III, thrombin and a clotting factor. However, in some embodiments a heparin which lacks anti-coagulant activity or which has reduced anti-coagulant activity may also be employed. Thus the heparin may have been modified so that it has from 0 to 80%, preferably from 5 to 60%, more preferably from 10 to 40% and even more preferably from 10 to 30% of the activity of the unmodified form or in comparison to unmodified heparin. Other glycosaminoglycans, in particular dermatan sulphate, also possess anticoagulant activity. Preferably, therefore, the glycosaminoglycans and their derivatives employed will retain some anti-coagulant activity, as discussed above for heparin and its derivatives.

As the glycosaminoglycan will be typically be administered via inhalation, intranasally or via installation preferably it will be in a form suitable for administration via such a route. In particular, the glycosaminoglycan may be in a form suitable for inhalation and/or installation.

### Subject Assessment

The present invention provides for the use of a glycosaminoglycan or a physiologically acceptable salt thereof in the manufacture of a medicament for facilitating the clearance of mucus from the central and peripheral airways of a human subject with chronic airflow limitation (CAL) who has mucus hypersecretion wherein the said glycosaminoglycan or salt has an average molecular weight of from 12 to 18 kilodaltons. The glycosaminoglycan or salt used, the route of delivery and any of the other parameters of the medicament and subject being treated may be the same as described herein for any of the other embodiments of the invention.

The medicaments of the invention preferably induce an improvement in the condition of the subject. The medicaments may therefore be used to manage a patient suffering from, or prone to, CAL. They may prevent, ameliorate, improve or cure the condition. They may slow down or arrest the progressive deterioration characteristic of CAL or in some cases even cause some reversal of the deterioration. They may prevent, reduce or reverse one or more of the symptoms and manifestations associated with CAL. They preferably will also increase the feeling of well being in the subject and their quality of life.

A medicament of the invention preferably reduces, eliminate or at least prevents further increase in one or more of:
- the elevated decline in forced expiratory volume (FEV₁);
- mucus hypersecretion;
- inflammation; and/or
- damage to the structure of the lung.

Treatment with the medicaments of the invention may also mean that the ratio of FEV₁ /FVC does not decline further, or is improved. For example, the ratio may be closer to that expected in a healthy subject.

The medicaments may reduce mucus plugging. They may have mucolytic activity and/or reduce secretion and increase expectoration of mucus. The medicaments of the invention facilitate the clearance of mucus and, due to their mucolytic activity, prevent or ameliorate detrimental effects associated with elevated mucus levels and increased viscosity in CAL. The medicaments of the invention may result in a reduction of mucus viscosity of, for example, at least one fold, preferably two fold and more preferably five fold. In some cases the decrease in viscosity may be from 10 to 80%, preferably from 20 to 60% and even more preferably from 30 to 40%.

Mucolytic activity refers to the reduction of viscoelasticity (viscosity) of mucus. Mucolytic activity may be determined by any of several different methods known in the art, including sputum compaction assay (see for example WO 94/10567) assays using a torsion pendulum (Janmey, J. Biochem. Biophys. Methods 22:41-53 1991) or other suitable rheological methodologies. The decrease in mucus viscosity brought about by the medicaments and methods of the invention may be referred to as mucokinesis.

The medicaments of the invention may reduce the breakdown of the structure of the lung, such as the degradation of elastin in the airways and in the aveoulus and hence the loss of lung elasticity. They may reduce or prevent the collapse of portions of the lung and/or the development of enlarged airspaces in which air can become trapped. The medicaments may prevent or reduce any of the pathological changes associated with CAL outlined herein. In particular, they may prevent progression of a pathological change. They may also prevent, or delay; the onset of a particular pathological change.

The medicaments of the invention may typically reduce the decline in FEV₁ by from 10 to 100%, preferably from 20 to 80%, more preferably from 30 to 60% and even more preferably from 40 to 50%. They may reduce the annual decline in FEV₁ by from 10 to 100 ml, preferably from 20 to 60 ml and even more preferably from 30 to 40ml per year. In some cases on treatment the subject will display an improvement of FEV₁ so that FEV₁ is from 25 to 100%, preferably from 40 to 100%, more preferably from 60 to 100% and even more preferably from 80 to 100% of the predicted value.

The medicaments of the invention may reduce inflammation and the influx of inflammatory cells to the airways and lungs. They may also ameliorate the effects of the inflammatory cells present in the lung. Thus differential cell counts on sputum and/or lavages from the subject may display normal levels, or less elevated levels, of inflammatory cells such as lymphocytes, macrophages and/or neutrophils and in particular of neutrophils.

They may reduce migration of inflammatory cells such as neutrophils to the airways and lung: They may also reduce one or more of neutrophil functions/characteristics such as priming, activation, chemotaxis, extravasation, degranulation, respiratory burst, phagocytosis, apoptosis and/or necrosis. This may also mean that there are reduced amount of neutrophil proteins in the lung and reactive oxygen species. There may also be reduced levels of inflammatory mediators in subjects following treatment and in particular of IL-8 levels. They may also cause changes in the lung such as the down regulation of adhesion molecules so that inflammatory cells and in particular neutrophils cannot enter the lung, do so less readily or do so in reduced numbers.

The medicaments of the invention may eliminate, delay the onset, or reduce the severity of any of the symptoms and features of CAL mentioned herein.

### Administration and formulation

The medicaments of the present invention may be prepared by formulating the glycosaminoglycan or salt with a standard physiologically, and in particular pharmaceutically, acceptable carrier and/or excipient as is routine in the pharmaceutical art. The exact nature of the formulation will depend upon several factors including the particular glycosaminoglycan employed and the desired route of administration. Suitable types of formulation are fully described in Remington's Pharmaceutical Sciences, 19th Edition, Mack Publishing Company, Eastern Pennsylvania, USA, the disclosure of which is included herein of its entirety by way of reference.

The necessary dose to be administered will normally be determined by a physician, but will depend upon a number of factors such as the nature of the condition to be treated, the condition of the patient. The dose of glycosaminoglycan administered may, for example, be from 0.01mg to 5g, preferably from 0.1 mg to 2.5g, more preferably from 1 mg to 1g, even more preferably from 10 mg to 500mg, still more preferably from 50 mg to 250 mg and even more preferably from 100 mg to 200 mg. These doses will typically be given once, twice or three times a day and will preferably be given once or twice a day and more preferably will be given twice a day.

For heparin and derivatives thereof and salts of either, dose will typically be in the range of from 10 to 10,000 units per/kg body weight, preferably 100 to 10,000 units per/kg body weight, more preferably from 200 to 5000 units/kg body weight, even more preferably from 500 to 2000 units per/kg, and still more preferably from 1,000 to 1,500 units per/kg. A unit of heparin activity (United States Pharmacopeia) is defined as the amount of heparin that prevents 1ml of citrated sheep plasma from clotting for one hour after adding 0.2 ml of 1% CaCl₂. These doses will typically be given once, twice or three times a day and will preferably be given twice a day.

The length of treatment may typically be from two weeks, a month, six months a year or more. In many cases the subject will remain on the medicaments of the invention permanently or for extended periods. This may in particular be the case where the subject does not stop smoking or continues to be exposed to the chemical pollutant thought to be the causative agent. This may also be the case where the subject has a genetic predisposition to developing CAL and most likely will need it indefinitely. The treatment schedule may also be coordinated so that at times when the CAL increases in severity such as times, or periods, of increased breathlessness and/or inflammation that the dose of glycosaminoglycan given is elevated or these may be the main time that glycosaminoglycan is administered. The medicament may be given prior to exercise or physical exertion and may typically be given as an aid to physiotherapy. It may be given during infections or when an infection is suspected.

Preferably the medicaments of the invention are administered via inhalation and/or intra-nasally. Thus administration may typically be via the mouth or nose. In an especially preferred embodiment, the medicaments of the invention are suitable for administration by inhalation and are administered via that route. The medicaments may be also be, or alternatively may be, suitable for administration via installation. Suitable methods for formulating and preparing medicaments to be administered via inhalation, installation and intranasally are well known in the art and may be employed in the present invention.

For inhalation therapy the medicament may be in a solution useful for administration by liquid aerosol, metered dose inhalers, or in a form suitable for a dry powder inhaler. The medicament may be present in a blister pack or breakable capsule.

In some preferred embodiments, the medicaments of the present invention may be formulated as aerosols. The formulation of pharmaceutical aerosols is routine to those skilled in the art, see for example, Sciarra, J. in Remington (*supra*). The agents may be formulated as solution aerosols, dispersion or suspension aerosols of dry powders, emulsions or semisolid preparations. The aerosol may be delivered using any propellant system known to those skilled in the art. The aerosols may be applied to the upper respiratory tract, for example by nasal inhalation, or to the lower respiratory tract or to both. The glycosaminoglycan may be delivered using liposomes and nano-particle delivery methods. Liposomes, particularly cationic liposomes, may be used in carrier formulations.

Medicaments for use in accordance with the present invention, may include, in addition to active ingredient, a pharmaceutically acceptable excipient, carrier, buffer, stabiliser or other materials well known to those skilled in the art. In particular they may include a pharmaceutically acceptable excipient. Such materials should be non-toxic and should not interfere with the efficacy of the active ingredient. The precise nature of the carrier or other material will depend on the route of administration. Suitable pharmaceutical carriers are described in Remington *(supra).*

The medicaments of the present invention may be delivered by any device adapted to introduce one or more therapeutic compositions into the upper and/or lower respiratory tract. In some preferred embodiments, the devices of the present invention may be metered-dose inhalers. The devices may be adapted to deliver the therapeutic compositions of the invention in the form of a finely dispersed mist of liquid, foam or powder. The device may use a piezoelectric effect or ultrasonic vibration to dislodge powder attached on a surface such as a tape in order to generate mist suitable for inhalation. The devices may use any propellant system known to those in the art including, but not limited to, pumps, liquefied-gas, compressed gas and the like.

In cases where the glycosaminoglycan is administered in the form of particles or droplets, the particle/droplet size and/or other properties of the particle/droplet may be chosen to ensure that the particles are delivered to a particular region of the respiratory tract. For example, they may be designed to reach only the upper or lower parts of the respiratory tract. In cases where the glycosaminoglycan, salt or therapeutic agent are delivered in an aqueous form preferably the solution will be isotonic to help ensure effective delivery to the subject. In particular, particles with a diameter of 10 µM are thought to be effective in reaching the lower parts of the respiratory tract and hence may be employed where such a site is the desired target for the medicaments. In embodiments, where it is desired to deliver the medicament to the lower parts of the respiratory tract, such as alveoli for example, the diameter of the particles administered may be less than 10 µM, preferably less than 8 µM, more preferably less than 6 µM and even more preferably less than 4 µM. In a preferred embodiment the particles may have a diameter of 3 µM or less and more preferably may have a diameter of 2µM or less. In an especially preferred embodiment the particles will have a diameter of from 3 to 5µM. In some cases the particles administered may be less than 1000 nm, preferably less than 500 nm, more preferably less than 250 nm and still more preferably less than 100 nm in diameter. The sizes may refer to particles of solid matter or droplets of solutions and suspensions.

The size of particles necessary to penetrate to a specific part of the respiratory tract will be known in the art and hence the particle size can be chosen to suit the target size. Techniques such as milling may be used to produce the very small particles necessary. In some cases the desired part of the respiratory tract may be the upper respiratory tract and hence larger particles sizes may be employed. The density of the particles and their shape may also be chosen to facilitate their delivery to the desired site.

The medicaments of the invention may take a variety of forms. They may be in the form of powders, powder microspheres, solutions, suspensions, gels, nano-particle suspensions, liposomes, emulsions or microemulsions. The liquids present may be water or other suitable solvents such as a CFC or HFA. In the case of solutions and suspensions these may be aqueous or involve solutions other than water.

Devices of the present invention typically comprise a container with one or more valves through which the flow of the therapeutic composition travels and an actuator for controlling the flow. Suitable devices for use in the present invention may be seen in, for example, Remington *(supra).* The devices suitable for administering the medicaments of the invention include inhalers and nebulisers such as those typically used to deliver steroids to asthmatics. In some cases, for example where the subject is a child, a spacer may be used in conjunction with the inhaler to help ensure effective delivery.

The medicaments of the present invention may be delivered by any device adapted to introduce one or more therapeutic compositions into the upper and/or lower respiratory tract. In some preferred embodiments, the devices of the present invention may be metered-dose inhalers. The devices may be adapted to deliver the therapeutic compositions of the invention in the form of a finely dispersed mist of liquid, foam or powder. The device may use a piezoelectric effect or ultrasonic vibration to dislodge powder attached on a surface such as a tape in order to generate mist suitable for inhalation. The devices may use any propellant system known to those in the art including, but not limited to, pumps, liquefied-gas, compressed gas and the like.

Devices of the present invention typically comprise a container with one or more valves through which the flow of the therapeutic composition travels and an actuator for controlling the flow. Suitable devices for use in the present invention may be seen, for example, in Remington's Pharmaceutical Sciences *(supra).* The devices suitable for administering the medicaments of the invention include inhalers and nebulisers such as those typically used to deliver steroids to asthmatics. In some cases, where the subject is for example a child, a spacer may be used to facilitate effective administration from the inhaler.

Various designs of inhalers are available commercially and may be employed to deliver the medicaments of the invention. These include the Accuhaler, Aerohaler, Aerolizer, Airmax, Autohaler, Clickhaler, Diskhaler, Easi-breathe inhaler, Fisonair, Integra, Jet inhaler, Miat-haler, Novolizer inhaler, Pulvinal inhaler, Rotahaler, Spacehaler, Spinhaler, Syncroner inhaler and Turbohaler devices. A number of formulation techniques which produce particularly desirable particles are known in the art and may be employed. For example the nanocrystal , pulmosol and pulmosphere technologies may be employed.

In some cases the medicaments may be administered via installation. In such cases, typically the medicament will be in liquid form and will be administered via an artificial airway such as, for example, an endotracheal tube. The liquid will typically be drawn up into a syringe and then expelled through the artificial airway into the respiratory tract of the subject. Installation is often used in an emergency context. In many cases it may be used where the subject has a relatively advanced form of CAL and has been admitted to hospital.

The medicaments of the invention may also be administered via the nose. Again, suitable methods, formulations and devices are known in the art for intranasal administration and may be employed. In cases where the medicament is administered via the nose the actual target site may be the nasal mucus membranes or alternatively some other part of the respiratory tract. The medicament will be typically formulated and administered accordingly. In cases where the medicament is to be administered via the nose it may be, for example, in the form of nasal spray. The spray may be administered, for example, using an atomizer or nebulizer. In some cases the medicament may be in the form of nasal drop. These may be administered using a via a medicine dropper. For further discussion on nasal dosage forms see Remington's Pharmaceutical Sciences (*Supra*). Medicaments administered via the nose may include pH adjusters, emulsifiers or dispersing agents, preservatives, surfactants, gelling agents, or buffering agents. Most preferably the nasal dosage form will be isotonic with nasal secretions.

The medicaments may include various constituents to optimise their suitability for the particular delivery route chosen. The viscosity of the medicaments may be maintained at a desired level using a pharmaceutically acceptable thickening agent. Thickening agents that can be used include methyl cellulose, xanthan gum, carboxymethyl cellulose, hydroxypropyl cellulose, carbomer, polyvinyl alcohol, alginates, acacia, chitosans and combinations thereof. The concentration of the thickening agent will depend upon the agent selected and the viscosity desired.

In some embodiments, and in particular where intranasal delivery is to be used, the medicaments may comprise a humectant. This may help reduce or prevent drying of the mucus membrane and to prevent irritation of the membranes. Suitable humectants include sorbitol, mineral oil, vegetable oil and glycerol; soothing agents; membrane conditioners; sweeteners; and combinations thereof.

The medicaments may comprise a surfactant. Suitable surfactants include non-ionic, anionic and cationic surfactants. Examples of surfactants that may be used include, for example, polyoxyethylene derivatives of fatty acid partial esters of sorbitol anhydrides, such as for example, Tween 80, Polyoxyl 40 Stearate, Polyoxy ethylene 50 Stearate, fusieates, bile salts and Octoxynol.

The following Examples illustrate the invention.

### Example 1: Barrier Assay on Victor Fluorometer

Sputum samples were thawed at room temperature and homogenised using a syringe (no needle). Samples were split and portions (0.12-0.20 g) weighed in small round-bottomed tubes. Tubes were centrifuged in a microfuge to sediment sputum. Mucolytic was added to sputum on a 10% volume to weight basis (e.g. 13 µl/0.13 g sputum). The mucolytic was therefore diluted by a factor of 10 upon addition to sputum. The mucolytics assessed were DNase,(at a concentration of 2.9 µg/ml of a stock of activity 2500 kunitz units per mg) and unfractionated heparin (at a concentration of 10 mg/ml of a stock of 160 USP/mg - a sodium heparin salt isolated from porcine intestinal mucosa from Calbiochem, catalogue number 375095, was employed). A control portion of sputum was treated with PBS on a 10% volume to weight basis. A further control was treated with dextran sulphate, which is not a glycosaminoglycan, at a concentration of 10 mg/ml (a sodium salt of dextran sulphate obtained from Sigma, catalogue number D7037, was employed). The mucolytic was thoroughly mixed with the sputum by vortexing and passing up and down a 19-gauge needle.

The lower wells of a Transwell chamber contained 25µl of phosphate buffered saline (PBS) and this was separated from the upper wells by an 8µm pore-size polycarbonate filter (placed shiny side down). The upper section of the chamber was placed on top of the filter and screwed down tightly. Carboxylate-modified fluorescent beads (F-8811, Molecular Probes) were sonicated for 5 mins to disperse them and beads added to sputum samples in a 1:1 ratio, e.g. 150µl beads added to 0.15g sputum sample. The mixture was vortexed to mix and 20µl of each sample added to the upper wells of the chamber. Each sample was added to the chamber in quadruplicate. The chamber was centrifuged for 5 mins at 1000 rpm to remove air bubbles, then placed inside a black plastic bag and incubated in a humid chamber at 37°C, 900rpm, for 4 hrs.

Following incubation, the chamber was centrifuged for 5 mins at 1000rpm to force migrated beads to the bottom of the lower wells. The upper chamber and gasket were removed in one piece to minimise leakage. The contents of the lower wells (25µl) were transferred to a Fluoro-NUNC plate and 175µl PBS added per well. The plate was read using Victor fluorometer with the 'Fluospheres' protocol (excitation 485nm, emission 535nm).

The results obtained are shown in the top panel of Figure 1. The mucolytic effects of heparin were greater and less variable than those of DNase. A Bonferri multiple comparisons test showed that the effect of 10 mg/ml heparin was significantly greater than that of the PBS control (P<0.01). However, the effects of DNase and 10 mg/ml dextran sulphate (negative control) were not significantly different when compared to the PBS control (P>0.05).

The effect of chondroitin sulphate on microsphere transport was also assessed using the same conditions as those described above. A 70:30 mixture of chondroitins A and C was tested (a sodium salt of the chondroitins from bovine trachea, obtained from Sigma, catalogue number C-8529, was employed). A control containing sputum treated with PBS alone was again run. The results obtained are shown in the bottom panel of Figure 1. Chondroitin sulphate was found to significantly increase transport of the microspheres.

### Example 2: Atomic Force Microscopy (AFM) imaging of DNA

Calf thymus DNA (0.1 mg/ml) was treated with heparin at various concentrations (0.1, 1, 10 and 100 µg/ml). Solutions were prepared in 0.2 µm filtered, DNase and RNase free water. 10 µl of sample was added to freshly cleaved ruby muscovite mica and dried prior to AFM imaging. AFM was performed in air under ambient conditions using a TopoMetrix TMX2000 Scanning Probe Microscope (ThermoMicroscopes, Bicester, UK) with a 70'70'12 mm tripod piezoelectric scanner. Topography measurements, in contact mode, were made using a "V"-shaped silicon nitride cantilever (length 200 nm, nominal spring constant (K) 0.21 Nm-1; Part. No. 1530-00, ThermoMicroscopes, Santa Clara, California, USA) bearing an integrated standard profile tip.

The results are shown in Figures 2 and Figure 3. In Figure 2, Panel A = 0.1 mg/ml DNA and Panel B = 0.1 mg/ml DNA + 0.1 mg/ml heparin. The addition of heparin to DNA was shown to alter the structure of the DNA network, increasing the average pore size from 180 (SD 30) µm to 780 (SD 150) µm.

Figure 3 shows results with lower concentrations of heparin. Panel A shows the results for untreated DNA: Panel B for DNA treated with 0.1µg/ml heparin; Panel C for DNA treated with 1 µg/ml heparin; and Panel D for DNA treated with 10 µg/ml heparin.

### Example 3: Patient Assessment

A female patient, age 81 years, with ALD (an ex-smoker, ceased 6 years ago) inhaled unfractionated heparin at a dose of 50,000 units, twice a day for 14 days. Although there was no change in spirometry over this short period, there was a marked improvement in symptoms of cough, and improved clearance of sputum.

## Claims

1. Use of a glycosaminoglycan or a physiologically acceptable salt thereof in the manufacture of a medicament for facilitating the clearance of mucus from the central and peripheral airways of a human subject with chronic airflow limitation (CAL) who has mucus hypersecretion wherein the said glycosaminoglycan or salt has an average molecular weight of from 12 to 18 kilodaltons and the medicament is administered via inhalation, intranasally, and/or via instillation.

2. Use according to claim 1, wherein the human subject has an FEV₁ of from 10 to 75% of the predicted value.

3. Use according to claim 1 or 2, wherein the subject is suffering from a bacterial or viral infection.

4. Use according to any one of the preceding claims, wherein the glycosaminoglycan or the physiologically acceptable salt comprises repeating disaccharide units of general formula (1)
-[A-B]- (1)
wherein:
- each A is the same or different and represents a moiety of formula (i) or (ii) 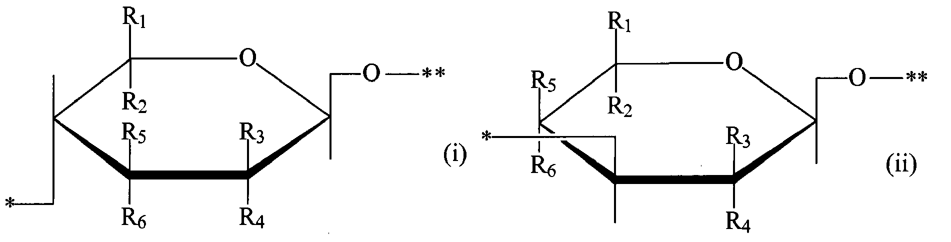
wherein:
- one of R₁ and R₂ is hydrogen, and the other is -CO₂H, -SO₃H or -CH₂OR wherein R is hydrogen or -SO₃H;
- one of R₃ and R₄ is hydrogen, and the other is -OR wherein R is hydrogen or -SO₃H;
- one of R₅ and R₆ is hydrogen, and the other is -OH;
- * represents a direct bond to an adjacent hydrogen atom or B moiety; and
- ** represents a direct bond to an adjacent B moiety;
each B is the same or different and represents a moiety of formula (iii) or (iv) 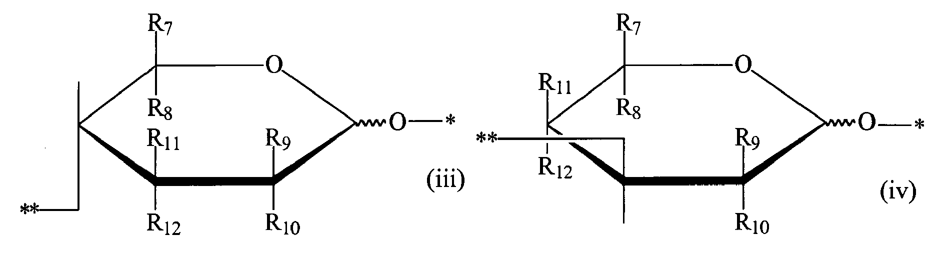 wherein:
- one of R₇ and R₈ is hydrogen and the other is -CH₂OH or -CH₂OSO₃H;
- one of R₉ and R₁₀ is hydrogen and the other is -NHAc, -NH₂ or -NHSO₃H;
- one of R₁₁ and R₁₂ is hydrogen and the other is -OH or -OSO₃H;
- 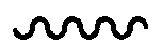 indicates a bond in either stereochemical orientation;
- * represents a direct bond to a hydrogen atom or an adjacent A moiety;
- ** represents a direct bond to an adjacent A moiety.
or a physiologically acceptable salt thereof.

5. Use according to any one of the preceding claims, wherein the sodium salt of heparin or heparin sulphate is used.

6. Use according to any one of the preceding claims, wherein the glycosaminoglycan or salt:
(a) has an average molecular weight of from 14 to 18 kd;
(b) has anti-coagulant activity; and/or
(d) has not been subjected to fragmentation and/or depolymerisation.

7. Use according to any one of the preceding claims, wherein the medicament is:
(a) for treating a subject who has a FEV₁ which is from 20 to 50% of the predicted value for an equivalent subject not suffering from CAL; and/or
(b) for use in reducing mucus plugging.

8. Use according to any one of the preceding claims, wherein the subject is, or has been, a smoker.

9. A glycosaminoglycan or a physiologically acceptable salt thereof for use in facilitating the clearance of mucus from the central and peripheral airways of a human subject with chronic airflow limitation (CAL) who has mucus hypersecretion wherein the said glycosaminoglycan or salt has an average molecular weight of from 12 to 18 kilodaltons and is administered via inhalation, intranasally, and/or via instillation.

## Patentansprüche

1. Verwendung von Glycosaminoglycan oder eines physiologisch annehmbaren Salzes davon bei der Herstellung eines Medikaments zur Erleichterung der Schleimbeseitigung der zentralen und peripheren Atemwege eines menschlichen Subjektes mit chronischer Luftströmungsbeschränkung (CAL), welcher eine Schleimübersekretion aufweist, wobei das Glycosaminoglycan oder Salz ein durchschnittliches Molekulargewicht von 12 bis 18 Kilodalton besitzt und das Medikament mittels Inhalation, intranasal und/oder via Instillation verabreicht wird.

2. Verwendung gemäß Anspruch 1,
wobei das menschliche Subjekt einen FEV₁-Wert von 10 bis 75% des vorausgesagten Wertes besitzt.

3. Verwendung gemäß Anspruch 1 oder 2,
wobei das Subjekt an einer bakteriellen oder viralen Infektion leidet.

4. Verwendung gemäß mindestens einem der vorhergehenden Ansprüche, wobei das Glycosaminoglycan oder das physiologisch annehmbare Salz davon wiederkehrende Disaccharideinheiten der folgenden allgemeinen Formel (1) umfasst
- [A - B] - (1)
worin
- jedes A gleich oder unterschiedlich ist und für einen Rest der Formel (i) oder (ii) steht 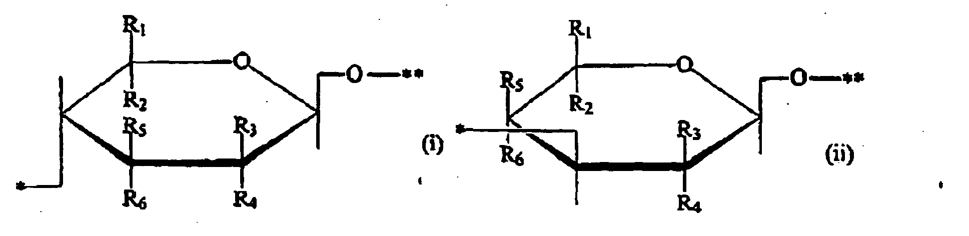
worin:
eines von R₁ oder R₂ Wasserstoff ist und das andere -CO₂H, -SO₃H oder - CH₂OR ist worin R Wasserstoff oder -SO₃H ist;
- eines von R₃ und R₄ Wasserstoff ist und das andere -OR ist, worin R Wasserstoff oder -SO₃H ist;
- eines von R₅ und R₆ Wasserstoff ist und das andere -OH ist;
- * eine direkte Bindung an ein benachbartes Wasserstoffatom oder B-Rest repräsentiert; und
- ** eine direkte Bindung an einen benachbarten B-Rest repräsentiert;
jedes B gleich oder verschieden ist und für einen Rest (iii) oder (iv) steht 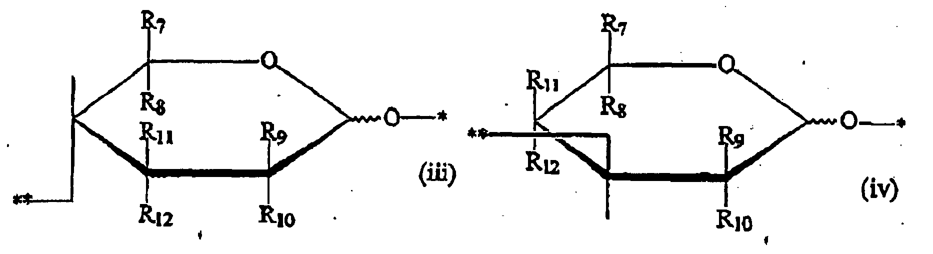 worin
- eines von R₇ und R₈ Wasserstoff ist und das andere -CH₂OH oder -CH₂OSO₃H ist;
- eines von R₉ und R₁₀ Wasserstoff ist und das andere -NHAc, -NH₂ oder - NHSO₃H ist;
- eines von R₁₁ und R₁₂ Wasserstoff ist und das andere -OH oder -OSO₃H ist;
- 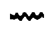 eine Bindung in jedweder stereochemischen Orientierung angibt;
- * eine direkte Bindung an ein Wasserstoffatom oder einen benachbarten A-Rest repräsentiert;
- ** eine direkte Bindung an einen benachbarten A-Rest repräsentiert;
oder ein physiologisch annehmbares Salz davon.

5. Verwendung gemäß mindestens einem der vorhergehenden Ansprüche, wobei das Natriumsalz von Heparin oder Heparinsulfat verwendet wird.

6. Verwendung gemäß mindestens einem der vorhergehenden Ansprüche, wobei das Glycosaminoglycan oder Salz:
(a) ein durchschnittliches Molekulargewicht von 14 bis 18 kd aufweist;
(b) Anti-Koagulans-Aktivität besitzt; und/oder
(c) keiner Fragmentierung und/oder Depolymerisation unterzogen worden ist.

7. Verwendung gemäß mindestens einem der vorhergehenden Ansprüche, wobei das Medikament:
(a) für die Behandlung eines Subjektes ist, welches einen FEV₁-Wert aufweist, welcher 20 bis 50% des vorausgesagten Wertes für ein äquivalentes Subjekt beträgt, das nicht an CAL leidet; und/oder
(b) zur Verwendung bei der Verringerung der Schleimverstopfung ist.

8. Verwendung gemäß mindestens einem der vorhergehenden Ansprüche, wobei das Subjekt ein Raucher ist oder gewesen ist.

9. Glycrosaminoglycan oder ein physiologisch annehmbares Salz davon zur Verwendung bei der Erleichterung der Schleimbeseitigung der zentralen und peripheren Atemwege eines menschlichen Subjektes mit chronischer Luftströmungsbeschränkung (CAL), welcher eine Schleimübersekretion aufweist, wobei das Glycosaminoglycan oder Salz ein durchschnittliches Molekulargewicht von 12 bis 18 Kilodalton besitzt und mittels Inhalation, intranasal und/oder via Instillation verabreicht wird.

## Revendications

1. Utilisation d'un glycosaminoglycane ou d'un sel physiologiquement acceptable de celui-ci dans la fabrication d'un médicament pour faciliter l'évacuation de mucus des voies aériennes centrales et périphériques d'un sujet humain avec une limitation chronique du débit d'air (CAL) qui a une hypersécrétion de mucus dans laquelle ledit glycosaminoglycane ou sel a un poids moléculaire moyen de 12 à 18 kilodaltons et le médicament est administré via inhalation, par voie intranasale, et/ou via instillation.

2. Utilisation selon la revendication 1, dans laquelle le sujet humain a un FEV₁ de 10 à 75% de la valeur prédite.

3. Utilisation selon la revendication 1 ou 2, dans laquelle le sujet souffre d'une infection bactérienne ou virale.

4. Utilisation selon l'une quelconque des revendications précédentes, dans laquelle le glycosaminoglycane ou le sel physiologiquement acceptable comprend des unités disaccharide répétées de formule générale (1)
-[A-B]- (1)
dans laquelle :
- chaque A est le même ou est différent et représente une fraction de formule (i) ou (ii) 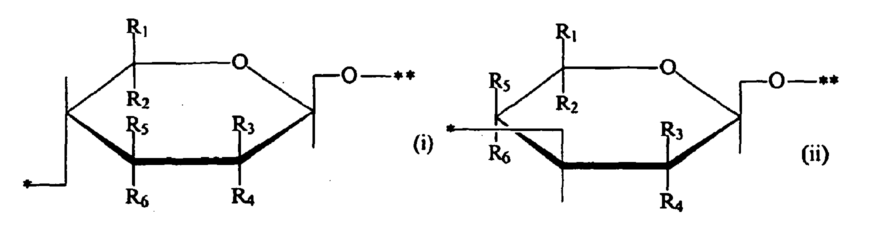
dans laquelle :
- un de R₁ et R₂ est de l'hydrogène, et l'autre est -CO₂H, -SO₃H ou -CH₂OR, R étant de l'hydrogène ou -SO₃H,
- un de R₃ et R₄ est de l'hydrogène et l'autre est -OR, R étant de l'hydrogène ou -SO₃H ;
- un de R₅ et R₆ est de l'hydrogène, et l'autre est -OH ;
- * représente une liaison directe à un atome d'hydrogène ou à une fraction B adjacent(e) ; et
- ** représente une liaison directe à une fraction B adjacente ;
chaque B est le même ou est différent et représente une fraction de formule (iii) ou (iv) 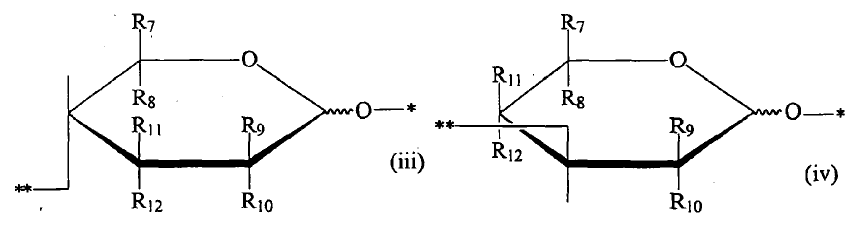 dans laquelle
- un de R₇ et R₈ est de l'hydrogène et l'autre est -CH₂OH ou -CH₂OSO₃H ;
- un de R₉ et R₁₀ est de l'hydrogène et l'autre est -NHAc, -NH₂ ou NHSO₃H,
- un de R₁₁ et R₁₂ est de l'hydrogène et l'autre est -OH ou -OSO₃H ;
- 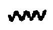 indique une liaison dans l'une ou l'autre orientation stéréochimique ;
- * représente une liaison directe à un atome d'hydrogène ou une fraction A adjacente ;
- ** représente une liaison directe à une fraction A adjacente
ou un sel physiologiquement acceptable de celui-ci.

5. Utilisation selon l'une des revendications précédentes, dans laquelle le sel de sodium d'héparine ou le sulfate d'héparine est utilisé.

6. Utilisation selon l'une des revendications précédentes, dans laquelle le glycosaminoglycane ou sel :
(a) a un poids moléculaire moyen de 14 à 18 kd ;
(b) a une activité anti-coagulante, et/ou
(c) n'a pas été soumis à une fragmentation et/ou une dépolymérisation.

7. Utilisation selon l'une des revendications précédentes, dans laquelle le médicament est :
(a) pour le traitement d'un sujet qui a un FEV₁ qui est de 20 à 50 % de la valeur prédite pour un sujet équivalent non souffrant de CAL ; et/ou
(b) pour l'utilisation dans la réduction d'obstruction par mucus.

8. Utilisation selon l'une des revendications précédentes, dans laquelle le sujet est, ou a été, un fumeur.

9. Glycosaminoglycane ou un sel physiologiquement acceptable de celui-ci pour l'utilisation dans la facilitation de l'évacuation de mucus des voies aériennes centrales et périphériques d'un sujet humain avec une limitation chronique du débit d'air (CAL) qui a une hypersécrétion de mucus dans laquelle ledit glycosaminoglycane ou sel a un poids moléculaire moyen de 12 à 18 kilodaltons et est administré via inhalation, par voie intranasale, et/ou via instillation.
